# EUROPEAN PATENT SPECIFICATION

(11) **EP 3 886 775 B1**
(45) Date of publication and mention of the grant of the patent: **19.08.2026**
(21) Application number: 19821439.7
(22) Date of filing: 29.11.2019
(51) Int. Cl.: A61F 5/058

(54) **UNIVERSAL SPLINT MODULE**
UNIVERSELLES SCHIENENMODUL
MODULE D'ATTELLE UNIVERSEL

(30) Priority: 29.11.2018 EE 201800030 U; 19.11.2019 EE 201900063 U
(43) Date of publication of application: 06.10.2021
(73) Proprietor: Ethr Oü, 10150 Tallinn (EE)
(72) Inventor: MASSO, Mihkel, 10920 Tallinn (EE); ARRO, Rait, 11622 Tallinn (EE)
(74) Representative: Renaudo, Adrien Hanouar
(86) International application number: PCT/EE2019/000001
(87) International publication number: WO 2020/108722

(56) References cited:
- WO-A1-2011/126365
- US-A- 2 486 687
- US-A1- 2001 022 183

## Description

### FIELD OF THE INVENTION

The present invention relates to a non-invasive medical equipment which enables supporting, fixing, and/or immobilising post-traumatic musculo-skeletal limb injuries.

The invention is intended for use by trauma medicine specialists of military and civil rescue services in complicated field conditions requiring the use of lightweight and compact splints enabling fast installation.

### BACKGROUND of the invention

Various special-purpose splints are known in the state of the art. Some of those are described below. More specifically, a cardboard splint is described which is the closest solution to the present invention in the state of the art.

### Vacuum splint

Vacuum splints are made of airtight composite textile panels, filled with small plastic pellets. Vacuum splints are wrapped around the injured limb and when the air is pumped out, the splint will harden. Vacuum splints come with various sizes and shapes, as the shape of each splint corresponds to the shape of the specific limb. Using such a solution requires a specific splint as well as a specific air pump. Pumping in field conditions is time-consuming. Vacuum splints do not function if the splint panel has been punctured. The drawbacks of this solution are its dimensions and the weight of the set of vacuum splints, which causes the need to always use some means of transport, and therefore, it is not suitable for complicated field conditions.

### Pneumatic splint

Pneumatic splints are made of airtight composite plastic panels. Pneumatic splints are wrapped around the injured limb and when the air is pumped in, the splint will harden. Pneumatic splints come with various sizes and shapes, as the shape of each splint corresponds to the shape of the specific limb. Using such a solution requires a specific splint as well as a specific air pump. Pumping in field conditions is time-consuming. Pneumatic splints do not function if the splint panel has been punctured. The drawback of this solution is low reliability in field conditions. Also, the pneumatic splint cannot ensure the structural rigidity needed for immobilizing bone injuries.

### Malleable sheet splint

Malleable sheet splints are typically made of an aluminium sheet covered with a thin layer of soft foam plastic. Sheet splints are bendable in multiple directions, which enables crafting various shapes and angles suitable for different shapes of limbs. The drawback of this solution is the low structural rigidity of sheet splints: during evacuation activities, the splint may bend or deflect, and cause secondary injuries to the injured limb. In practice, sheet splints are suitable only for splinting metacarpus, the wrist, and the ankle joint.

### Wire splint

Wire splints are made of bendable wire and their shape resembles a ladder. Contemporary wire splints are surrounded by a soft padding made of cellular plastic and film wrapping material. Wire splints are bendable, which enables crafting various shapes and angles suitable for different shapes of limbs. Using this solution requires a special wire-cutting tool and plenty of time. The drawback of this solution is the difficult material: when using a wire splint, it is difficult to change the already bent shape. In practice, it is difficult for a trauma medicine specialist to carry along a wire splint in field conditions due to its dimensions.

### Field-expedient splint

Several armed forces worldwide teach the students of combat medicine to craft a splint of handy materials found in field conditions. Typically, such improvised splints consists of tape and flat wooden slats. The splint is fixed around the injured limb with triangular bandages or strips torn from the clothes of the casualty. The correct use of an improvised splint requires extensive special training and suitable component materials in field conditions. The drawback of this solution is also the very long time needed for making and installing the splint.

### Traction splint

Traction splints are devices enabling the application of traction or longitudinal pulling force on the muscles of the lower limb. The device does not enable the fixation of the lower limb in the lateral direction. The use of traction splints is indicated only in case of a femur injury. Although a traction splint can be folded in the centre, the device is still very large and awkward to use in field conditions.

### Cardboard splint

Cardboard splints are known as the closest solution to the present invention.

Cardboard splints are made of cardboard or nowadays also of three-layer corrugated plastic. Cardboard splints include a base panel supporting the injured limb and side panels stabilizing the limb. The panels are mutually laterally connected with at a 90° angle. When using a cardboard splint, the limb of the injured person must be fixed inside the splint with plenty of padding material, as the splint does not follow the anatomic shape of the injured limb and the injured limb can start moving in the gap between the limb and the splint, which may cause secondary injuries. Furthermore, splints with a different length should be carried along to splint injuries of low or upper limbs.

A drawback of cardboard splints is its insufficient structural rigidity, poor pliability into U- or C-shape. In order to find a suitable size, the medical personnel must carry along many splints with various sizes. Another drawback of cardboard splints is the material itself, which may easily degrade upon contact with moisture or water.

In conclusion, although the category of splints is broadly covered, there is still no splint that would be suitable for demanding field conditions. Such a solution should be light, compact, and ensure the structural rigidity for external fixation. The solution should also enable splinting most or all common types of musculo-skeletal injuries and be suitable for injured persons with different anatomic parameters. This would enable preventing carrying along several different types and sizes of splints. The installed splint must ensure orthopedically correct embracing of the injured limb in a comfortable manner for the user to ensure good ventilation and minimize the risk of pressure ulcers.

International patent application WO2011/126365 relates to splint for an injured limb, which splint can be placed against the injured limb and which consist of at least one blank of compressed paper fibres. The splint consists of at least two separate splint elements, which can be connected together using connecting means.

### Summary of the invention

The object of the invention is to provide a universal splint module for post-traumatic splinting of musculo-skeletal limb injuries, where:
- the identical shape of splint sheets and the identical location of fixing elements enables to create a splinting functionality from similar splint sheets for fixing any different possible musculo-skeletal limb injuries of human casualties;
- folding grooves of the splint sheet and their location enable configuring the width and length dimensions of the splint to be orthopedically suitable for the anatomic dimensions of the limbs of different human casualties;
- identical shape of splint sheets, identical location of fixing elements, and folding grooves of splint sheet and their location enable easy supplement and/or replacement of splint sheets as part of a splint set;
- the identical shape of splint sheets, the identical location of fixing elements, and the folding grooves of the splint sheet and their location enable packaging the device into a compact transport and package position;
- the folding grooves and folding apertures of the splint sheet and their location enable folding back the tip of the material sheet, thus creating a support with an orthopedically neutral angle for splinting metacarpus and the wrist;
- the folding grooves and folding apertures of the splint sheet and their location enable bending fixing wings out of the material sheet for splinting the injured lower limb of a human casualty, the corners of which retain the fixing or binding means fixing the device around the limb.

All the above listed properties form complete objects of the invention, which can be met with a single technical solution, i.e. the proposed universal splint module for post-traumatic splinting of all musculo-skeletal limb injuries.

The present invention proposes a universal splint module comprising a material sheet with folding grooves and folding apertures and fixing elements, which enable joining several universal splint modules for making special-purpose splinting functionalities.

A universal splint module is basically a rectangular sheet with horizontal, vertical folding grooves. Folding grooves can also be diagonal or angular. The location of folding grooves enables configuring the width and length of the splint into an orthopedically suitable size for the limb of the specific casualty.

The location of the fixing elements of the universal splint module enables joining the modules with each other. Joining the modules enables forming splints with various sizes and functionalities for splinting most musculo-skeletal limb injuries.

The fixing elements of a splint module can be rivets, dowels, snaps, tenons, swivels etc. The fixing elements are homogenous with the sheet or added separately. The fixing elements may join the splint sheets with each other as follows:
1. with the lower ends or the lower and upper ends of the universal splint module into an L-shaped splint, where splint sheets form a U-shape;
2. with the lower ends, or lower or upper ends, or upper ends of the universal splint module, where splint sheets are located on each other in a U-shape, enabling forming an extended splint.

By folding the upper part of the universal splint module back along the upper folding line, a metacarpus support can be formed to achieve an orthopedically neutral support angle needed for splinting metacarpus and the wrist.

The universal splint module has fixing lugs, which can be bent outwards and the corners of which retain the fixing or binding means fixing the device around the limb.

The location of folding grooves of the universal splint module furthermore enables packaging the splint into a compact transport and package position.

### List of illustrations

The above mentioned and other properties and advantages of the invention are described more in detail below together with references to the appended figures, which illustrate the preferred embodiments, where
figure 1 is the view of the universal splint module from above;
figure 2 is a general view of a wrist splinted with the universal splint module;
figure 3 illustrates a forearm, elbow joint, and humerus splinted with the universal splint module;
figure 4 illustrates an ankle joint splinted with the universal splint module;
figure 5 illustrates a tibia and knee joint splinted with the universal splint module;
figure 6 illustrates a femur splinted with the universal splint module;

### Example of an embodiment

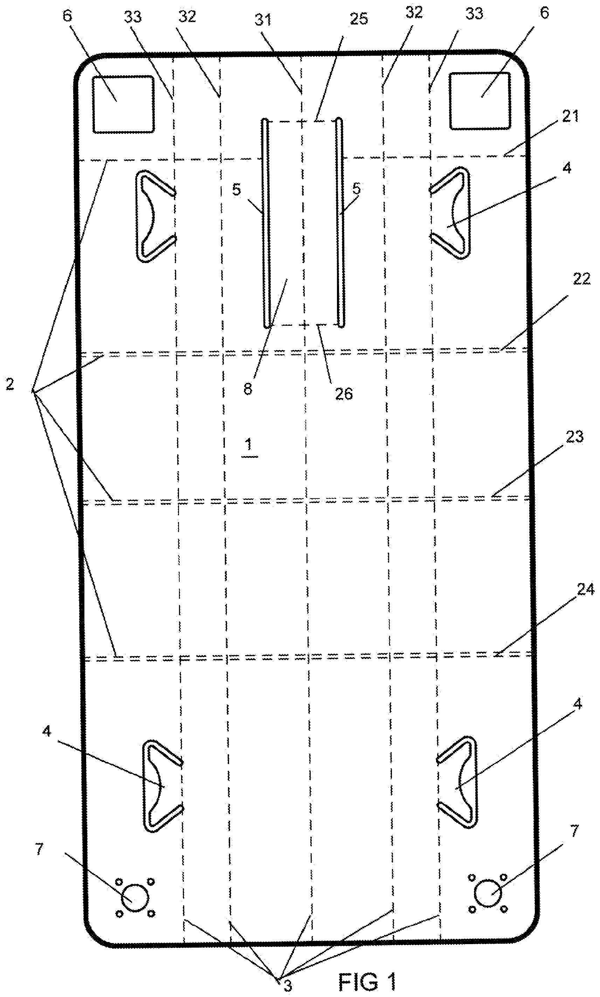

Figure 1 illustrates splint sheet 1 of the universal splint module with visible horizontal recessed grooves 2 and vertical recessed grooves 3, which facilitate folding the splint. Splint sheet 1 has fixing wings 4, which can be bent out, and cut-outs 5. Splint sheet 1 has apertures 6 and 7 for locating the fixing elements to form a splint from splint sheets 1 of the universal splint module. Splint sheet 1 has wrist and metacarpus support 8, which can be bent out.

### Shape and construction of a sheet

The shape of splint sheet 1 consists of a rectangular basic form. The upper or shorter edge of the rectangle is 20-40 cm long. The height of the rectangle is 40-60 cm.

All corners of the outer contour (inner as well as outer corners) are rounded with a radius of 0.01-25 mm.

### Recessed lines

The recessed lines in the splint sheet are straight grooves with a depth of 0.1 mm to ¾ thickness of the sheet on the top and bottom surface of the sheet.

Horizontal recessed lines 2 perpendicular to the longitudinal axis of the sheet or folding lines extending over the entire splint sheet are following:
21 At the distance of 3-8 cm from the upper edge of splint sheet 1 over the entire splint sheet, except for the wrist support in the centre of the splint sheet;
22 At the distance of 13-25 cm from the upper edge of splint sheet 1 or approximately one third of the total length over the entire splint sheet;
23 At the distance of 20-29 cm from the upper edge of splint sheet 1 or in the central part of the splint sheet over the entire splint sheet;
24 At the distance of 13-25 cm from the lower edge of splint sheet 1 or approximately one third of the total length over the entire splint sheet.
25 At the distance of 2-4 cm from the upper edge of splint sheet 1, between the upper edge and recessed line 21, extending 3-4 cm on both sides of the longitudinal axis.
26 At the distance of 16-18 cm from the upper edge of splint sheet 1, between recessed lines 21 and 22 closer to recessed line 22, extending 2-4 cm on both sides of the longitudinal axis.

Vertical recessed lines 3 extending over the entire splint sheet:
31 On the longitudinal axis of the splint sheet 1;
32 On both sides of the longitudinal axis of the splint sheet 1, 3-7 cm from the longitudinal axis;
2.3. At the distance of 3-8 cm from the outer longitudinal edges of the splint sheet 1 at the side of the longitudinal axis.

### Cut-outs

Splint sheet 1 has straight or curved cut-outs 5 extending mirrored in relation to the longitudinal axis. The cut-outs start from the ends of recessed line 25 and end at the ends of recessed lines 26, forming wrist and metacarpus support 8. The cut-outs are 0.1-3 mm wide.

Splint sheet 1 has C-shaped or trapezoidal cut-outs starting from recessed lines 33, which form fixing wings 4 when bent out, whereby the parallel sides of the trapezoid are vertical at +- 10 degrees; the shorter side of the trapezoid is located on recessed line 33 and the cut-out extends only along the legs and longer side of the trapezoid; and the corners of the trapezoid may be rounded. The legs of the trapezoid or the highest and the lowest point of the C-shaped cut are located between recessed lines 21 and 22, 1-8 cm from each line. The cut-outs extend from recessed lines 33 with an outward direction from the longitudinal axis. The longer side of the trapezoid may be rounded towards the longitudinal axis of the splint sheet.

The upper part of splint sheet 1 includes quadrangular, rectangular, or circular cut-outs located 0.5-5 cm from the edges of the sheet, which form apertures 6 for fixing details.

The lower side of the splint sheet 1 includes quadrangular, rectangular, or circular cut-outs located 0.5-5 cm from the edges of the splint sheet, which form apertures 7 for fixing details.

### Fixing elements

Splint sheet 1 has fixing elements 9, which are rivets, snaps, loops, buttons, eyelets, swivels, etc. Fixing elements 9 are inserted into relevant apertures 6, 7 at the upper and lower edge of splint sheet 1. Fixing elements enable joining splint sheets 1 of the universal splint module with each other to obtain a splint with required length.

### Splint sheet

Splint sheet 1 is made of thin sheet material, which can be cellulose-based, thermoplastic, plastic, sandwich, composite, etc. material. Splint sheet 1 may be transparent or opaque. The material may be slightly flexible in longitudinal, transverse, and other directions. The material can also be composite or sandwich material, the different layers of which have different properties, e.g. an upper layer or layers with cut-outs of non-flexible material, and flexible intermediate or cover layer, which would ensure similar spatial behaviour e.g. in the case of a polypropylene sheet with thinned bending lines. The relation between the location of bending lines and the properties of the material defines the final flexibility of splint sheet 1. The sheet may include, partially or to the full extent, softening elements, e.g. a neoprene layer, which ensures the better compatibility of the sheet with the splinted limb during splinting. The bending of the splint sheet around the limb around the longitudinal axis of splint sheet 1 defines the final rigidity of splint sheet 1 as a splint. Splint sheet 1 may have apertures for aeration or for providing support for bandage or other fixing elements in various locations. Splint sheets may have different cut-outs in different locations, which form different shapes upon folding to ensure better support to the injured limb. The thickness of splint sheets can be from 0.1 mm to 5 mm, preferably 2 mm. Splint sheets can have different sizes, e.g. 60 × 25 cm. The length of splint sheets can be changed by folding at recessed lines 2, 3 or elsewhere. Furthermore, splint sheets can be cut mechanically with cutting means.

### Use

### Usage scenario 1: Infantry squad/platoon

A fire contact is made with an adversary. The fire contact results in a casualty. Contemporary typical injury mechanisms: handgun fire (bullet), indirect fire (mine, shell, rocket, grenade fragment), explosion (mine fragment, shooting foreign objects from an improvised explosive, as well as air overpressure, throwing the casualty against surrounding objects).

### Stage (1): Care Under Fire

The battle buddy of the casualty provides life-saving first aid to the casualty near the contact line, i.e. installs tourniquet on the limb and/or opens airways, if necessary. If the tactical situation allows, i.e. the suppressive fire laid down by the casualty's unit suppresses the adversary, so that handgun fire is no more efficient, the casualty is evacuated by dragging away from the area of influenced by direct small arms fire of the adversary. The distance depending on the landscape and tactical situation is approximately 25-150 meters. For dragging, the battle buddy grabs the accoutrements/bulletproof vest or clothes of the casualty. If possible, the conscious casualty must assist dragging as much as possible.

### Stage (2): Tactical Field Care

The combat paramedic of the squad or platoon level provides life-saving stabilising first aid to the casualty. The physician operates based following the CABCDE or MARCH(E) (from slide no. 13) algorithm. Time is limited (1-10 min) and at any moment, a change in the tactical situation may cause the need to terminate all operations based on an algorithm, and evacuate the casualty immediately to the next stage of aid.

In the CABCDE or MARCH(E) stage, the ETHR universal splint modules will be used. The combat paramedic takes universal splint modules 1 from their medical bag, opens vacuum package, and places splint module 1 beside the casualty.

The combat paramedic must decide the necessary configuration of the splint of splint sheets 1 of the universal splint module. They take splint modules 1 needed for the required configuration and join them with fixing elements 9. The combat paramedic checks the suitability of the length and width of the splint on the uninjured limb of the casualty. If necessary, the combat paramedic makes the splint smaller/shorter or larger/longer.

Usually, two pairs of hands are required for splinting. One keeps the injured limb in a fixed position. The other installs and fixes the splint onto the injured limb. The rule of 'two neighbours' is followed during fixing the splint. I.e., in the case of a bone injury, the joints located on both sides of the bone are fixed; in the case of a joint injury, the bones located on both sides of the joint are fixed.

The splint is fixed around injured limb with triangular bandages, dressing, elastic bandage, tape, elastic tape, self-adhesive tape, Velcro strip, other fixing straps with fixing elements at the ends (e.g. fast fixing snaps, buckets, eyes) or strips torn from the clothes of the casualty.

If the time and tactical situation allow it, the physician must perform a secondary examination based on the BATLS algorithm. During this examination, the physician checks if the splint has been correctly fixedc and if a distal pulse can be detected (i.e. on the wrist, behind the ankle joint).

When the casualty has been splinted, the casualty will be placed into an evacuation device or onto an evacuation stretcher. The immobile casualty will be rolled onto the bag by using the 'single piece method'. If the height position of the casualty must be changed (i.e. in relation to the vertical axis of the evacuation device), the 'diagonal sawing method' is used. The casualty can stay on the evacuation device lying on the back or in the 'coma position', i.e. permanently on the side.

The combat paramedic organises the transport or lifting of the casualty with the help of the other combatants of the subunit of the casualty. Usually, the combat paramedic does not evacuate the casualty to the next aid stage themselves. The casualty is taken by dragging or carrying to the Ambulance Exchange Point.

### Stage (3): Tactical Evacuation

The casualty has been evacuated to the Ambulance Exchange Point. Here, the casualty must be loaded onto the evacuation vehicle. The Ambulance Exchange Point must have an armed guard, but may not have a physician, who would handle actively the casualty until the arrival of the evacuation vehicle.

In an ideal situation, the casualty and the evacuation vehicle reach the Ambulance Exchange Point at the same time. However, in practice, the casualty often waits the evacuation vehicle in a 'packaged state'. Such a delay is caused by the tactical situation and long distances, as well as the overload of evacuation vehicles. During this waiting period, the decrease of body temperature becomes especially dangerous to the health condition of the casualty (a decrease of the body temperature accelerates the weakening of blood coagulation factors). If the time and tactical situation allow it, the primary and secondary examination will be repeated.

Upon the arrival of the evacuation vehicle, the casualty is handed over to the accepting party. In the course of this procedure, the MIST (Mechanism of injury, Injuries, Signs and symptoms, Treatments) report card or combat casualty card is read out loud, the casualty is lifted onto the stretcher of the vehicle and then onto the evacuation vehicle.

Evacuation vehicles are divided into two categories: MEDEVAC (Medical Evacuation, i.e. a medical evacuation vehicle with clear marking and a team with special skills and equipment) and CASEVAC (Casualty Evacuation, i.e. any evacuation vehicle without marking and which may lack a team with special skills and equipment). CASEVAC vehicles often lack temperature control, i.e. there is a risk that the body temperature of the casualty may decrease further.

Upon the arrival of the MEDEVAC vehicle, a trained combat paramedic or combat lifesaver will receive the casualty at the Ambulance Exchange Point. They must monitor the casualty during tactical evacuation. If necessary, the receiving person will repeat the primary examination during transport based on the CABCDE or MARCH(E) algorithm.

Upon the arrival of the CASEVAC vehicle, a person without special skills must receive the casualty (e.g. the driver or another person). They have no skills for intervention if the condition of the casualty worsened at the stage of tactical evacuation.

The evacuation vehicle takes the casualty to get medical help of the next level. The destination may vary depending on the injury and health parameters of the casualty, as well as on the tactical situation and logistical capabilities. The options are: Role 0 Casualty Collection Point or Forward Medical Post, Role 1 Batallion Aid Station, Role 2 Brigade Field Hospital, or Role 3 Combat Support Hospital or a regional or central hospital in a civil hospital system.

Not depending on the aid level, when the casualty reaches the next aid level with tactical evacuation, the receiving operations are the same. The arrival of the evacuation vehicle transporting the casualty has been generally announced in advance over the radio. The evacuation vehicle arrives at the vehicle reception area.

The transferor reads out the MIST report or combat casualty card of the casualty to a member of the triage team of the receiving party. The member of the triage team must decide based on the MIST or combat casualty card to which department the casualty must be transferred (typically, the casualty is first transferred to the triage area or to the emergency room if spare resources are available, but rarely directly to the operating theatre).

The casualty is unloaded from the vehicle, loaded from the stretcher of the vehicle to the stretcher of the receiving aid level institution, and then taken to the department specified by the triage team member.

The transferor moves together with the casualty to the relevant department and reads there once more out loud the MIST or combat casualty card of the casualty to the receiving person of the department. The transferor answers any detailed questions. After that, the transferor is allowed to return to the vehicle, and leaves. Here, the stage of tactical evacuation ends.

In the receiving department, the primary (based on the CABCDE or MARCH(E) algorithm) and secondary examinations (based on the BATLS algorithm) are repeated.

When the examination of the casualty has been completed in the receiving department, the casualty may be transferred to emergency medical, operating, intensive care, or nursing department. In the receiving department, an X-ray radiograph is taken with a mobile X-ray device. This will be the basis for the decision if the limb injury of the casualty can be fixed on the spot with a permanent solution (gypsum splint) or the casualty must be evacuated to a higher aid level.

In the operating department, the splint modules can be removed so that a surgeon can fix the bone injury.

Used splint modules are discarded to waste together with other disposable medical supplies. Waste is collected and disposed (typically by incineration on high temperature).

In the transport position, the splint sheet is folded together. This is possible due to recessed lines 1 of the splint, located transversely to the splint.

In practice, the field physician uses folded splint modules for compact storage and transport in their field bag.

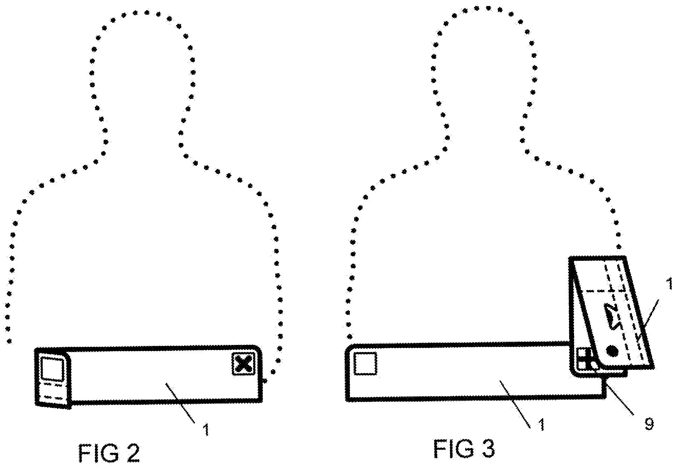

Figure 2 illustrates a universal splint module rolled into a C- or U-shape in relation to the longer axis used for splinting a joint. Another option is a universal splint module rolled into a C- or U-shape in relation to the longer axis, whereby a curved wrist support is formed in the upper part of the sheet by folding back the edge.

In practice, the field physician uses a single splint module for fixing small injuries, e.g. injuries of the metacarpus or the wrist. If the injury requires the supporting of the metacarpus (e.g. a stable fracture of the wrist joint), the physician decides to use curved wrist support 8 of the splint module. If the character of the injury does not require supporting the metacarpus or if the nature of the injury excludes supporting the metacarpus (e.g. a blunt trauma of the metacarpus), the physician decides not to use curved wrist support 8 of the splint module.

Figure 3 illustrates 'two universal splint modules joined at the lower parts of the sheet and rolled into a C- or U-shape. As another option, two universal splint modules are joined through the lower part of the sheet, whereby one universal splint module is folded shorter with the folding line of the closer axis located on the sheet.

In practice, the field physician decides to use two or more splint modules if major injuries need to be splinted, in the case of which the longitudinal dimension of a single module may not be sufficient. Or, the functionality of a single module is not sufficient for fixing the injury.

The configuration of two splint modules illustrated in figure 3, where the modules are joined at an angle through the lower parts, is suitable for fixing bone or soft tissue injuries of the forearm, elbow joint, humerus, and humeral joint.

If the physician has decided to use two splint modules, they must assess which is the best possible way for correcting the dimensions of the joined splint modules to ensure that the limb of the casualty is compactly and firmly surrounded and supported by the joined splint modules.

In this case, the physician has decided to correct the module surrounding the humerus by folding it back from the side facing the humeral joint so that the folded edge of the splint module fits comfortably under the armpit of the casualty while providing correct support to the area of humerus.

The physician has also decided to apply anatomically neutral wrist support 8. If the forearm of the casualty is longer than the length of the splint module modelled based on the average length of the forearm of a male person, so that the wrist and metacarpus of the casualty extend over the location of anatomically neutral wrist support 8 or over the edge of the splint module, the module supporting forearm and wrist can be extended with a third splint module. The excess length of the extension module can be corrected by folding back the unconnected side of the extension module.

When the physician has prepared the splint modules in a suitable configuration, they must check the suitability of the configuration on the uninjured limb of the casualty. If necessary, the dimensions of joined splint modules can be additionally corrected longitudinally as well as laterally by folding or cutting with scissors.

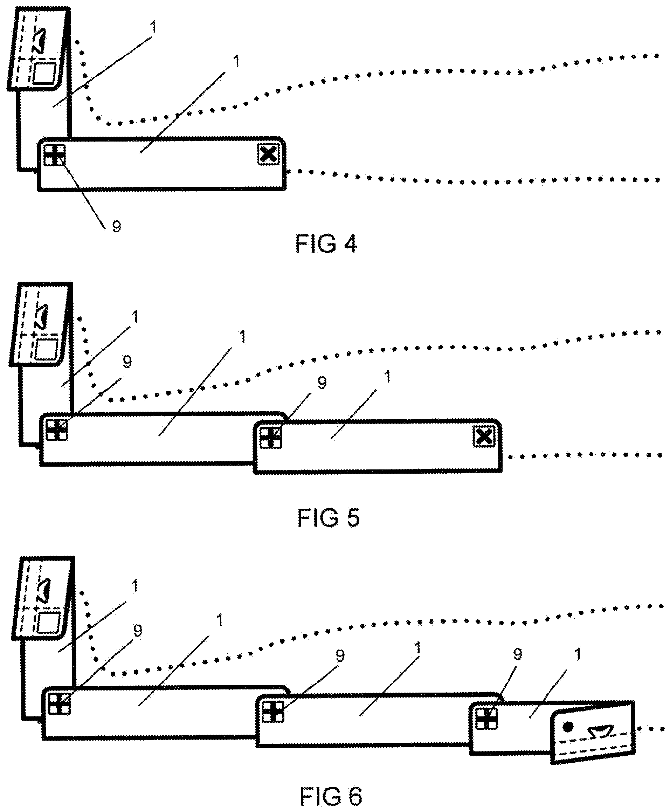

Figures 4 and 5 illustrate two universal splint modules joined into an L-shape through the lower part of the universal splint module. As an option, one universal splint module can be added, which is folded shorter with the folding line of the closer axis located on splint sheet 1, and the third splint sheet 1 is fixed to the upper end of the full-length sheet.

In practice, the field physician decides to use two or more splint modules if major injuries need to be splinted, in the case of which the longitudinal dimension of a single module may not be sufficient. Or, the functionality of a single module is not sufficient for fixing the injury.

In practice, the field physician decides to use the configuration of 'two splint modules illustrated in figure 4 in the case of injuries of the ankle joint and foot. An L-shaped configuration of the splint modules ensures anatomically neutral support for fixing the foot and ankle joint. In such a case, the physician must measure the length of the prepared configuration of splint modules on the uninjured limb of the casualty.

Cut-outs of fixing wing 4 can be bent out from the back folded part of the splint module fit under the foot. Bandage, tape, and other bandaging material fixing the configuration of the splint modules to the foot and ankle joint can be fastened behind the corners of these cut-outs.

In practice, the field physician decides to use the configuration extended with a third splint module illustrated in figure 5 in the case of injuries of the tibia or knee joint. This ensures the fixing of the foot and ankle joint as well as the leg and knee joint. In a special case with a very tall casualty, the configuration of the three splint modules can be extended with a fourth splint module to ensure the reach of the splint over the knee joint to half of the femur. If necessary, the dimensions of the fourth module can be additionally corrected by folding or cutting with scissors.

Figure 6 illustrates two universal splint modules joined through the lower part of the sheet, whereby one splint sheet 1 is folded shorter with the folding line of the closer axis located on the sheet, and third splint sheet 1 is fixed to the upper end of full-length sheet 1, while the fourth splint sheet 1 is fixed to its lower end, which is folded shorter with horizontal folding lines, if necessary.

In practice, the field physician decides to use the configuration extended with a fourth splint module illustrated in figure 6 in the case of a femur injury. In such a case, the physician must measure the length of the prepared configuration of splint modules on the uninjured limb of the casualty. It is important to ensure that the splint module reaches over the pelvic bone of the casualty to the hip. The excess length of the extension module can be folded back or cut off with scissors.

In the case of femoral splint, the splint is first fixed to the injured limb. Then, the injured splinted limb is fixed to the uninjured limb of the casualty with additional material enabling bandaging. This operation enables preventing the pivoting movement of the ends of the injured femur during evacuation in relation to each other, and thus minimise pain and the risk of secondary injuries. As an additional recommended measure, a pelvic splint can be installed to the casualty, which helps to fix pelvis and hip joints, and thus minimise pain and the risk of secondary injuries.

## Claims

1. Universal splint module for post-traumatic splinting of musculo-skeletal limb injuries, comprising a splint sheet (1) with recessed lines (2, 3), cut-outs (5), and apertures (6, 7) for fixing elements (9), which enable joining several splint sheets to generate splinting functionalities for various purposes, and **characterised in that** the splint sheet (1) has a rectangular shape with rounded corners having a radius of 0.01-25 mm, shorter edges of 20-40 cm and a height of 40-60 cm, and further comprises a wrist and metacarpus support (8), wherein at least 4 horizontal recessed lines (2) are extending over the entire splint sheet (1) at the exception of the wrist and metacarpus support (8) area and are respectively located at 3-8 cm (21) from the upper edge of the splint sheet (1), 13-25 cm (22) from the upper edge of the splint sheet (1), 20-29 cm (23) from the upper edge of the splint sheet (1), and 13-25 cm (24) from the lower edge of the splint sheet (1), wherein at least 5 vertical recessed lines (3) are extending over the entire splint sheet (1) and located respectively on the longitudinal axis (31) of the splint sheet (1), 3-7 cm (32) on both sides of said longitudinal axis (31), and 3-8 cm (33) from both outer longitudinal axis, wherein the splint sheet (1) has 2 additional horizontal recessed lines respectively located at 2-4 cm (25) from the upper edge of the splint sheet (1), between the upper edge and the first horizontal recessed line (21), and extending 3-4 cm on both sides of the longitudinal axis (31), and 16-18 cm (26) from the upper edge of the splint sheet (1), between the first (21) and second (22) horizontal recessed line, and extending 2-4 cm on both sides of the longitudinal axis, and wherein the cut-outs (5) start from the end of the horizontal recessed line (25) and end at the end of the horizontal recessed line (26), forming the wrist and metacarpus support (8).

2. Universal splint module according to claim 1, **characterised in that** the splint sheet (1) has 4 horizontal recessed lines (2) extending over the entire splint sheet (1) at the exception of the wrist and metacarpus support (8) area and are respectively located at 3-8 cm (21) from the upper edge of the splint sheet (1), 13-25 cm (22) from the upper edge of the splint sheet (1), 20-29 cm (23) from the upper edge of the splint sheet (1), and 13-25 cm (24) from the lower edge of the splint sheet (1), and 5 vertical recessed lines (3) extending over the entire splint sheet (1) and located respectively on the longitudinal axis (31) of the splint sheet (1), 3-7 cm (32) on both sides of said longitudinal axis (31), and 3-8 cm (33) from both outer longitudinal axis.

3. Universal splint module according to claim 1 or 2, **characterised in that** the splint sheet (1) has at least one C-shaped or trapezoidal cut-out starting from one of the two vertical recessed line (33) and forming a fixing wing (4), which can be bent outwards.

4. Universal splint module according to claims 3, **characterised in that** the shorter side of the trapezoid of the trapezoidal cut-out is located on a vertical recessed line (33) and the cut-out extends only along the legs and longer side of the trapezoid.

5. Universal splint module according to claims 1-4, **characterized in that** the fixing elements (9) are part of the splint sheet (1).

6. Universal splint module according to claims 1-5, **characterised in that** the fixing elements (9) are swivels, rivets, snaps, eyelets, buttons or loops.

7. Universal splint module according to claims 1-6 for use as a non-invasive medical equipment for supporting, fixing and immobilising post-traumatic musculo-skeletal limb injuries.

8. Universal splint module for use according to claim 7, wherein sheets of the universal splint module are joined together with fixing elements (9) for forming splints with various sizes and functionalities for splinting musculo-skeletal limb injuries.

9. Universal splint module for use according to claim 7 or 8, wherein sheets of the universal splint module are rolled into a C- or U-shape in relation to the longer axis to form a splint supporting the limb.

10. Universal splint module for use according to claim 8 or 9, **characterised in that** the required length of the splint is achieved by adding additional sheets, which are connected to each other and/or by joining the splint sheets with each other and by folding back or cutting the splint sheet (1) at relevant horizontal recessed line (2).

## Patentansprüche

1. Universelles Schienenmodul zur posttraumatischen Schienung von Verletzungen des Bewegungsapparats der Extremitäten, umfassend eine Schienenplatte (1) mit Vertiefungslinien (2, 3), Ausschnitten (5) und Öffnungen (6, 7) für Befestigungselemente (9), die das Verbinden mehrerer Schienenplatten ermöglichen, um Schienenfunktionen für verschiedene Zwecke zu erzeugen, **dadurch gekennzeichnet, dass** die Schienenplatte (1) eine rechteckige Form mit abgerundeten Ecken mit einem Radius von 0,01-25 mm, kürzere Kanten mit einer Länge von 20-40 cm und eine Höhe von 40-60 cm aufweist und ferner eine Handgelenk- und Mittelhandstütze (8) umfasst, wobei sich mindestens vier horizontale Vertiefungslinien (2) mit Ausnahme des Bereichs der Handgelenk- und Mittelhandstütze (8) über die gesamte Schienenplatte (1) erstrecken und jeweils in einem Abstand von 3-8 cm
(21) vom oberen Rand der Schienenplatte (1), 13-25 cm (22) vom oberen Rand der Schienenplatte (1), 20-29 cm (23) vom oberen Rand der Schienenplatte (1) und 13-25 cm (24) vom unteren Rand der Schienenplatte (1) angeordnet sind, wobei sich mindestens fünf vertikale Vertiefungslinien (3) über die gesamte Schienenplatte (1) erstrecken und jeweils auf der Längsachse (31) der Schienenplatte (1), in einem Abstand von 3-7 cm (32) auf beiden Seiten der Längsachse (31) sowie in einem Abstand von 3-8 cm (33) von den beiden äußeren Längsachsen angeordnet sind, wobei die Schienenplatte (1) zwei zusätzliche horizontale Vertiefungslinien aufweist, die jeweils in einem Abstand von 2-4 cm (25) vom oberen Rand der Schienenplatte (1), zwischen dem oberen Rand und der ersten horizontalen Vertiefungslinie (21), angeordnet sind und sich auf beiden Seiten der Längsachse (31) über 3-4 cm erstrecken beziehungsweise in einem Abstand von 16-18 cm (26) vom oberen Rand der Schienenplatte (1), zwischen der ersten (21) und der zweiten (22) horizontalen Vertiefungslinie, angeordnet sind und sich auf beiden Seiten der Längsachse über 2-4 cm erstrecken, und wobei die Ausschnitte (5) am Ende der horizontalen Vertiefungslinie (25) beginnen und am Ende der horizontalen Vertiefungslinie (26) enden und dadurch die Handgelenk- und Mittelhandstütze (8) bilden.

2. Universelles Schienenmodul nach Anspruch 1, **dadurch gekennzeichnet, dass** die Schienenplatte (1) vier horizontale Vertiefungslinien (2) aufweist, die sich mit Ausnahme des Bereichs der Handgelenk- und Mittelhandstütze (8) über die gesamte Schienenplatte (1) erstrecken und jeweils in einem Abstand von 3-8 cm (21) vom oberen Rand der Schienenplatte (1), 13-25 cm (22) vom oberen Rand der Schienenplatte (1), 20-29 cm (23) vom oberen Rand der Schienenplatte (1) und 13-25 cm (24) vom unteren Rand der Schienenplatte (1) angeordnet sind, sowie fünf vertikale Vertiefungslinien (3), die sich über die gesamte Schienenplatte (1) erstrecken und jeweils auf der Längsachse (31) der Schienenplatte (1), in einem Abstand von 3-7 cm (32) auf beiden Seiten dieser Längsachse (31) sowie in einem Abstand von 3-8 cm (33) von den beiden äußeren Längsachsen angeordnet sind.

3. Universelles Schienenmodul nach Anspruch 1 oder 2, **dadurch gekennzeichnet, dass** die Schienenplatte (1) mindestens einen C-förmigen oder trapezförmigen Ausschnitt aufweist, der von einer der beiden vertikalen Vertiefungslinien (33) ausgeht und einen Befestigungsflügel (4) bildet, der nach außen gebogen werden kann.

4. Universelles Schienenmodul nach Anspruch 3, **dadurch gekennzeichnet, dass** die kürzere Seite des Trapezes des trapezförmigen Ausschnitts auf einer vertikalen Vertiefungslinie (33) liegt und sich der Ausschnitt nur entlang der Schenkel und der längeren Seite des Trapezes erstreckt.

5. Universelles Schienenmodul gemäß den Ansprüchen 1 bis 4, **dadurch gekennzeichnet, dass** die Befestigungselemente (9) Teil der Schienenplatte (1) sind.

6. Universelles Schienenmodul gemäß den Ansprüchen 1 bis 5, **dadurch gekennzeichnet, dass** die Befestigungselemente (9) Drehgelenke, Nieten, Druckknöpfe, Ösen, Knöpfe oder Schlaufen sind.

7. Universelles Schienenmodul gemäß den Ansprüchen 1 bis 6 zur Verwendung als nicht-invasives medizinisches Hilfsmittel zur Stützung, Fixierung und Ruhigstellung von posttraumatischen Verletzungen des Bewegungsapparats an den Gliedmaßen.

8. Universelles Schienenmodul zur Verwendung gemäß Anspruch 7, wobei die Platten des universellen Schienenmoduls mit Befestigungselementen (9) miteinander verbunden werden, um Schienen mit unterschiedlichen Größen und Funktionen zur Schienung von Verletzungen des Bewegungsapparats der Extremitäten zu bilden.

9. Universelles Schienenmodul zur Verwendung gemäß Anspruch 7 oder 8, wobei die Platten des universellen Schienenmoduls in Bezug auf die Längsachse zu einer C- oder U-Form gerollt werden, um eine die Extremität stützende Schiene zu bilden.

10. Universelles Schienenmodul zur Verwendung gemäß Anspruch 8 oder 9, **dadurch gekennzeichnet, dass** die erforderliche Länge der Schiene durch Hinzufügen weiterer, miteinander verbundener Schienenplatten und/oder durch Zusammenfügen der Schienenplatten sowie durch Zurückklappen oder Abschneiden des Schienenplatten (1) an einer entsprechenden horizontalen Vertiefungslinie (2) erreicht wird.

## Revendications

1. Module universel d'attelle pour l'attelle post-traumatique de lésions musculo-squelettiques des membres, comprenant une feuille d'attelle (1) comportant des lignes en retrait (2, 3), des découpes (5), et des ouvertures (6, 7) destinées à des éléments de fixation (9), qui permettent d'assembler plusieurs feuilles d'attelle afin de générer des fonctionnalités d'attelle pour diverses utilisations, et **caractérisé en ce que** la feuille d'attelle (1) présente une forme rectangulaire avec des coins arrondis ayant un rayon de 0,01 à 25 mm, des côtés les plus courts de 20 à 40 cm et une hauteur de 40 à 60 cm, et comprend en outre un support de poignet et de métacarpe (8), dans lequel au moins 4 lignes horizontales en retrait (2) s'étendent sur toute la feuille d'attelle (1), à l'exception de la zone du support de poignet et de métacarpe (8), et sont respectivement situées à 3 à 8 cm (21) du bord supérieur de la feuille d'attelle (1), à 13 à 25 cm (22) du bord supérieur de la feuille d'attelle (1), à 20 à 29 cm (23) du bord supérieur de la feuille d'attelle (1), et à 13 à 25 cm (24) du bord inférieur de la feuille d'attelle (1), dans lequel au moins 5 lignes verticales en retrait (3) s'étendent sur toute la feuille d'attelle (1) et sont situées respectivement sur l'axe longitudinal (31) de la feuille d'attelle (1), à 3 à 7 cm (32) de part et d'autre dudit axe longitudinal (31), et à 3 à 8 cm (33) des deux axes longitudinaux extérieurs, dans lequel la feuille d'attelle (1) comporte 2 lignes horizontales en retrait supplémentaires situées respectivement à 2 à 4 cm (25) du bord supérieur de la feuille d'attelle (1), entre le bord supérieur et la première ligne horizontale en retrait (21), et s'étendant sur 3 à 4 cm de part et d'autre de l'axe longitudinal (31), et à 16 à 18 cm (26) du bord supérieur de la feuille d'attelle (1), entre la première (21) et la deuxième (22) ligne horizontale en retrait, et s'étendant sur 2 à 4 cm de part et d'autre de l'axe longitudinal, et dans lequel les découpes (5) commencent à l'extrémité de la ligne horizontale en retrait (25) et se terminent à l'extrémité de la ligne horizontale en retrait (26), formant le support de poignet et de métacarpe (8).

2. Module universel d'attelle selon la revendication 1, **caractérisé en ce que** la feuille d'attelle (1) comporte 4 lignes horizontales en retrait (2) s'étendant sur toute la feuille d'attelle (1), à l'exception de la zone du support de poignet et de métacarpe (8), et étant respectivement situées à 3 à 8 cm (21) du bord supérieur de la feuille d'attelle (1), à 13 à 25 cm (22) du bord supérieur de la feuille d'attelle (1), à 20 à 29 cm (23) du bord supérieur de la feuille d'attelle (1), et à 13 à 25 cm (24) du bord inférieur de la feuille d'attelle (1), et 5 lignes verticales en retrait (3) s'étendant sur toute la feuille d'attelle (1) et situées respectivement sur l'axe longitudinal (31) de la feuille d'attelle (1), à 3 à 7 cm (32) de part et d'autre dudit axe longitudinal (31), et à 3 à 8 cm (33) des deux axes longitudinaux extérieurs.

3. Module universel d'attelle selon la revendication 1 ou 2, **caractérisé en ce que** la feuille d'attelle (1) comporte au moins une découpe en forme de C ou trapézoïdale partant de l'une des deux lignes verticales en retrait (33) et formant une ailette de fixation (4), qui peut être pliée vers l'extérieur.

4. Module universel d'attelle selon la revendication 3, **caractérisé en ce que** le côté le plus court du trapèze de la découpe trapézoïdale est situé sur une ligne verticale en retrait (33) et la découpe s'étend uniquement le long des côtés et du côté le plus long du trapèze.

5. Module universel d'attelle selon les revendications 1 à 4, **caractérisé en ce que** les éléments de fixation (9) font partie de la feuille d'attelle (1).

6. Module universel d'attelle selon les revendications 1 à 5, **caractérisé en ce que** les éléments de fixation (9) sont des pivots, des rivets, des boutons-pression, des œillets, des boutons ou des boucles.

7. Module universel d'attelle selon l'une quelconque des revendications 1 à 6, destiné à être utilisé comme équipement médical non invasif pour soutenir, fixer et immobiliser des lésions musculo-squelettiques des membres post-traumatiques.

8. Module universel d'attelle destiné à être utilisé selon la revendication 7, dans lequel des feuilles du module universel d'attelle sont assemblées au moyen d'éléments de fixation (9) afin de former des attelles présentant diverses tailles et fonctionnalités pour l'attelle de lésions musculo-squelettiques des membres.

9. Module universel d'attelle destiné à être utilisé selon la revendication 7 ou 8, dans lequel des feuilles du module universel d'attelle sont enroulées en une forme en C ou en U par rapport à l'axe le plus long afin de former une attelle soutenant le membre.

10. Module universel d'attelle destiné à être utilisé selon la revendication 8 ou 9, **caractérisé en ce que** la longueur requise de l'attelle est obtenue par ajout de feuilles supplémentaires, qui sont reliées les unes aux autres et/ou par assemblage des feuilles d'attelle les unes avec les autres et par repliement ou découpe de la feuille d'attelle (1) au niveau de la ligne horizontale en retrait (2) appropriée.
